# EUROPEAN PATENT SPECIFICATION

(11) **EP 1 267 817 B1**
(45) Date of publication and mention of the grant of the patent: **01.03.2006**
(21) Application number: 01925496.0
(22) Date of filing: 23.03.2001
(51) Int. Cl.: A61K 8/34, A61K 8/43, A61Q 11/00, A61K 8/49, A61K 8/60

(54) **ORAL COMPOSITION COMPRISING CHLORHEXIDINE AND MALTITOL OR ERYTHRITOL OR A MIXTURE THEREOF**
ORALE ZUBEREITUNG CHLORHEXIDIN UND MALTITOL ODER ERYTHRITOL ODER DEREN MISCHUNG ENTHALTEND
COMPOSITION ORALE CONTENANT DE LA CHLORHEXIDINE ET DU MALTITOL, DE L'ERYTHRITE OU UN MELANGE DESDITS COMPOSES

(30) Priority: 30.03.2000 GB 0007760
(43) Date of publication of application: 02.01.2003
(73) Proprietor: SMITHKLINE BEECHAM PLC, Brentford, Middlesex TW8 9EP (GB)
(72) Inventor: ALEXANDER, Stephen Edward, Weybridge, Surrey KT13 0DE (GB); MCCONVILLE, Peter Scott, Weybridge, Surrey KT13 0DE (GB); PORTMANN, Marie, Weybridge, Surrey KT13 0DE (GB)
(74) Representative: Reeves, Julie Frances
(86) International application number: PCT/EP2001/003255
(87) International publication number: WO 2001/074323

(56) References cited:
- EP-A- 0 934 742
- WO-A-99/16470
- WO-A-99/33352
- US-A- 4 279 888
- US-A- 4 601 900
- DATABASE CHEMABS [Online] CHEMICAL ABSTRACTS SERVICE, COLUMBUS, OHIO, US; OKA, HIRONORI: "Shellac-based tooth-coating compositions containing basic amino acids and pH controllers" retrieved from STN Database accession no. 132:156565 XP002175719 & JP 2000 044422 A (JAPAN) 15 February 2000 (2000-02-15)

## Description

The present invention relates to oral hygiene compositions having reduced bitterness which comprise the cationic antibacterial agent, chlorhexidine.

Cationic antibacterial agents such as chlorhexidine have long been recognised as useful therapeutic agents for treating various oral health conditions such as plaque, caries, periodontal diseases and halitosis due not only to their intrinsic in vitro antibacterial activity but also because of their substantivity to oral tissue. They are thus adsorbed onto oral surfaces and gradually released over a period of time, to produce a prolonged antibacterial effect and hence reduce or prevent the formation of plaque over a period of time. It is believed that the deposition of plaque onto the tooth surface is one of the first steps in the development of dental caries and periodontal disease.

In view of the extremely bitter taste of chlorhexidine, oral hygiene compositions comprising it are generally unpleasant to taste, unless the bitter taste can be effectively masked.

One method of masking the taste of chlorhexidine is described in US Patent 4601900. This document describes a mouthwash composition comprising from 0.1 to 0.2 % by weight of chlorhexidine together with from 2 to 15 % by weight of xylitol as the bitterness inhibitor.

WO 9933352 discloses chewing gum compositions comprising chlorhexidine digluconate as preferred antimicrobial agent and comprising also sugar alcohols like e.g. maltitol and xylitol.

WO9916470 discloses an oral administration preparation, in which unpleasant tastes of drugs are improved by jointly adding a sugar alcohol having a heat of dissolution of -20 cal/g or less, like e.g. erythritol, xylitol, mannitol and sorbitol.

Surprisingly it has now been discovered that maltitol or erythritol are more effective than xylitol in masking the bitterness of chlorhexidine.

Accordingly in a first aspect the present invention provides an oral hygiene composition comprising chlorhexidine or an orally acceptable addition salt thereof, a bitterness inhibitor selected from maltitol or erythritol or a mixture thereof, and an ionic fluorine-containing compound plus an orally acceptable carrier or excipient.

Suitably, chlorhexidine or an orally acceptable acid additive salt thereof is present in from 0.005 to 10 % , preferably 0.01 to 5 % , more preferably 0.01 to 2 % , by weight of the oral hygiene composition.

Suitable such salts of chlorhexidine include those which have a solubility at 20°C of at least 0.005% w/v and include the digluconate, diformate, diacetate, dipropionate, dihydroiodide, dihydrochloride, dilactate, dinitrate, sulphate, and tartrate. Of these, the digluconate, diacetate and dihydrochloride are favoured.

Suitably the bitterness inhibitor is present from 0.1 to 30 % , preferably from 1 to 20%, by weight of the oral hygiene composition. Typically 10 to 20% by weight in a toothpaste, and 2 to 6 % by weight in a mouthwash.

Preferably the bitterness inhibitor is maltitol.

Oral hygiene compositions of the present invention may also usefully contain an ionic fluorine-containing compound. Suitable ionic fluorine-containing compounds include, for instance, fluoride salts such as amine fluorides and alkali metal fluoride salts, for example sodium fluoride, and monofluorophosphate salts such as alkali metal monofluorophosphate salts, for example sodium monofluorophosphate. Suitably the ionic fluorine-containing compound is incorporated into the composition to provide between 100 and 3000ppm, preferably between 500 and 2000ppm of fluoride ions.

Oral hygiene compositions of the present invention may be provided in any of the presentations normally used for such products, for instance, dentifrices including toothpastes and toothpowders, abrasive and non-abrasive gels, mouthwashes, gargles, irrigating solutions, mouthsprays and presentations for sucking or chewing by the user such as gums, pastilles and lozenges. Components for the orally acceptable carrier or excipient will be selected according to the particular type of presentation involved.

It will be appreciated by those skilled in the art that in order to ensure that the antibacterial efficacy of the cationic antibacterial agent is not substantially diminished, compatible components will be selected for inclusion in the orally acceptable carrier or excipient. Accordingly, the skilled man will readily appreciate that where necessary anionic species should be preferably avoided as such species may cause inactivation of the cationic antibacterial agent by the formation of an insoluble precipitate therewith. Thus, anionic surfactants and anionic thickening agents should preferably be rejected in favour of non-anionic counterparts such as nonionic, cationic or amphoteric surfactants and nonionic thickening agents. Similarly, abrasives capable of acting as a source of anions, either because they are sparingly soluble or whilst being essentially insoluble per se are contaminated with soluble impurities arising from, for instance, the manufacture thereof, are preferably avoided in favour of compatible abrasives.

Suitable nonionic surfactants include, for example, polyethoxylated sorbitol esters, in particular polyethoxylated sorbitol monoesters, for instance, PEG(40) sorbitan di-isostearate, and the products marketed under the trade name 'Tween' by ICI; polycondensates of ethylene oxide and propylene oxide (poloxamers), for instance the products marketed under the trade name 'Pluronic' by BASF-Wyandotte; condensates of propylene glycol; polyethoxylated hydrogenated castor oil, for instance, cremophors; and sorbitan fatty esters.

Suitable amphoteric surfactants include, for example, long chain imidazoline derivatives such as the product marketed under the trade name 'Miranol C2M' by Miranol; long chain alkyl betaines, such as the product marketed under the tradename 'Empigen BB' by Albright + Wilson, and long chain alkyl amidoalkyl betaines, such as cocamidopropylbetaine, and mixtures thereof.

Suitable cationic surfactants include the D,L-2-pyrrolidone-5-carboxylic acid salt of ethyl-N-cocoyl-L-arginate, marketed under the trade name CAE by Ajinomoto Co. Inc.

Advantageously, the surfactant is present in the range 0.005 to 20%, preferably 0.1 to 10 % , more preferably 0.1 to 5% by weight of the dentifrice.

Suitable nonionic thickening agents include, for example, (C1-6)-alkylcellulose ethers, for instance methylcellulose, hydroxy(C1-6)-alkylcellulose ethers, for instance hydroxypropylcellulose, (C2-6)-alkylene oxide modified (C1-6)-alkylcellulose ethers, for instance hydroxypropyl methylcellulose, and mixtures thereof. Advantageously the nonionic thickening agent is present in the range 0.01 to 30%, preferably 0.1 to 15%, more preferably 1 to 5 % , by weight of the composition.

Suitable abrasives include a sparingly soluble salt, in combination with an agent to suppress anion formation, or an essentially insoluble compound.

Suitable sparingly soluble salts that may be used as an abrasive include calcium carbonate, calcium phosphates, magnesium carbonate, insoluble sodium metaphosphate, and suitable mixtures thereof. The agent to suppress anion formation typically comprises a water soluble salt containing a cation which may be same as the cation of the abrasive and which forms an essentially insoluble or sparingly soluble salt with the anion of the abrasive. Preferably the sparingly soluble salt used as an abrasive is calcium carbonate, advantageously used in combination with dicalcium phosphate, which also usefully buffers the pH of the formulation. Suitable types of calcium carbonate include both natural and synthetic chalks. The agent to suppress anion formation may be an alkaline earth metal salt, for instance calcium chloride. The agent is preferably present in from 0.0001 to 1 %, more preferably 0.005 to 0.1 % by weight of the dentifrice.

The term 'essentially insoluble compound' as used herein refers to a compound which is intrinsically insoluble in aqueous solution and includes those compounds which are listed as being 'insoluble' in cold water in the 'Handbook of Chemistry and Physics', 48th Edition, Chemical Rubber Company, Section B, Physical Constants of Inorganic Compounds. Furthermore, such compounds when used as an abrasive shall contain little if any contaminating anionic impurities. Preferably the insoluble abrasive compound should contains less than 1 %, preferably less than 0.5 % , and more preferably less than 0.25% of anionic impurities, based on the weight of the abrasive. Suitable essentially insoluble compounds for use as abrasives include, for example, silica, zinc orthophosphate, plastics particles, alumina, hydrated alumina, and calcium pyrophosphate or mixtures thereof.

Preferably, the abrasive is silica. Suitable silicas include natural amorphous silica, for instance diatomaceous earth; and synthetic amorphous silicas, for instance a precipitated silica, or a silica gel, such as a silica xerogel; or mixtures thereof. The preferred synthetic amorphous silicas are those with a low-level of water soluble anionic impurities (hereinafter referred to as "low-anion silica"). These are obtainable from manufacturing process which are carefully controlled so that the level of anion impurities, particularly sulphate and silicate from sodium sulphate and sodium silicate, respectively, is kept to a minimum. Alternatively, or in addition, the level of anion impurities may be reduced to the required level by careful washing of the initially produced silica with, for instance, deionised or distilled water. Suitable low-anion silicas contain less than 0.5 % , preferably less than 0.25 %, more preferably less than 0.1% by weight of water soluble impurities such as sodium sulphate and/or sodium silicate.

The abrasive is advantageously present in the range 1 to 80 % , preferably 5 to 70%, more preferably 5 to60% by weight of the dentifrice.

It will be appreciated that each of the thickening agent, the surfactant and abrasive should be, at the level employed in the dentifrice, compatible with the cationic antibacterial agent, that is, each will not substantially reduce the availability of the cationic antibacterial agent, for instance, by more than 30 % , preferably more than 20%. This may be confirmed by, for instance, determining the biological activity of the formulation, by conventional microbiological assay using, for instance, M. luteus as the assay organism in a standard agar diffusion method, in the presence and absence of each of the aforementioned thickening agent, surfactant and abrasive.

Mouthwashes according to the present invention will preferably comprise as components of the carrier a surfactant and a humectant in an aqueous or an aqueous/ethanol solution. Gels according to the present invention will preferably comprise as components of the carrier a surfactant, humectant, thickening agent and optionally water. Dentifrices according to the present invention will preferably comprise as components of the carrier a surfactant, humectant, thickening agent, abrasive and if necessary, water.

Suitable humectants for use in compositions of the invention include for instance glycerine, sorbitol, propylene glycol or polyethylene glycol, or mixtures thereof. The humectant may be present in the range from 5 to 70%, preferably 5 to 30%, more preferably 10 to 30% by weight of the dentifrice.

Other materials may be added to the compositions if required, for instance sweetening agents, flavouring agents, colouring and whitening agents, preservatives and emulsifiers.

Examples of sweetening agents include saccharin and salts thereof, cyclamate salts, acesulfame, aspartame, thaumatin, and sugar alcohols such as sorbitol and xylitol.

If desired, additional bitterness inhibitors may be added, for example sodium salts such as the chloride, bicarbonate, propionate, acetate, aspartate or gluconate.

The oral hygiene compositions of the present invention may also comprise an agent to inhibit the staining caused by chlorhexidine, for example polyvinylpyrrolidone as described in WO 9316681.

The pH of a composition according to the invention will be orally acceptable and typically in the range pH 5 to 9.

Oral hygiene compositions according to the present invention may be prepared by mixing the ingredients thereof in the required proportions and in any order that is convenient and thereafter and if necessary adjusting the pH to the required value.

In a further aspect, the present invention provides for an oral hygiene composition as hereinbefore defined for use in therapy. The present invention also provides for the use of chlorhexidine or an acid addition salt thereof and a bitterness inhibitor selected from maltitol or erythritol or a mixture thereof, in the manufacture of a composition for use in oral hygiene.

The invention will now be illustrated by reference to the following examples.

### Example 1: Masking the bitterness of chlorhexidine

The efficacy of maltitol, erythritol and xylitol to mask the bitter taste of chlorhexidine digluconate (CHXG) was evaluated using a panel of trained taste assessors.

All test solutions were made up in still mineral water. All tasting sessions were carried out at room temperature (20°C). Samples were made up w/v (unless otherwise specified) one day before tasting and stored in the refrigerator overnight, then allowed to equilibrate to room temperature the next day before serving.

Eighteen panellists volunteered for the study. The panellists were first screened for their taste acuity using basic identification ranking techniques. 0.20% sodium chloride, 0.054% citric acid, 0.8% sucrose, 0.04% caffeine and 0.15% potassium alum were used to determine their ability to sense and identify saltiness, sourness, sweetness, bitterness and astringency, respectively. The detection and identification thresholds for bitterness were then measured using eight increasing concentrations of quinine hydrochloride (over a range of 0 - 0.0012%). Panellists were also instructed to rank four random concentrations of caffeine in increasing order of bitterness intensity. A similar experiment was done using quinine hydrochloride. All panellists performed well in those tasks.

The screened panellists were then asked to assess the bitterness intensities of the chlorhexidine mixtures using a "Labelled magnitude line scale" (LMS) as defined by Green et al., Chemical Senses, 1996, 21, 323-334. Solutions were made up containing the bitter inhibitors, 0.1% w/w CHXG, 8% w/w ethanol in water. The following concentrations of inhibitors were used:

| | | | |
|---|---|---|---|
| Xylitol | 2 | & | 4% w/w |
| Maltitol | 2 | & | 4% w/w |
| Erythritol | 2 | & | 4% w/w |

Two samples were presented at any one time, one of which was the reference (0.1 % CHXG in 8 % ethanol-water). Sample presentation was randomised and was duplicated during the course of the study.

The "sip-and-spit" method was used for tasting CHXG solutions. Panellists were instructed to sip 5ml sample, swirl it around their mouth and spit it out immediately before judging its bitterness intensity. This technique was expected to take 3-4 seconds.

Panellists were instructed to taste the reference first and memorise its bitter intensity. This intensity was assigned the number 70 on the line scale marked 0 - 100. Panellists were asked to wait until they could no longer feel any bitterness in their mouths (approximately 10 min) before tasting the second solution, whose intensity they then ranked in proportion to the intensity of the reference on the same line scale. Panellists were provided with mineral water to cleanse their palates.

Analysis of Variance (ANOVA) at the 5 % level of significance was performed using the EXCEL software to find out whether there were any significant differences between the CHXG reference and the CHXG/inhibitor mixtures. A Fisher Least Significant Difference (LSD) test (O'Mahony M, 1986, Sensory Evaluation of Food - Statistical Methods and Procedures, Marcel Dekker, Inc, New York) was carried out on those samples where a significant difference had been found, to identify which sample means differ from the reference.

All the inhibitors tested were effective at suppressing the bitterness of CHXG. Analysis of variance (n = 18) has shown that the maltitol or erythritol mixtures are significantly different from the 0.1% CHXG reference, whose bitterness was set at 70 on the line scale. The percentage suppression achieved by each of the CHXG/inhibitor mixtures studied is shown below.

| Inhibitor used | % suppression of bitterness of 0.1 % CHXG |
|---|---|
| Xylitol 2 % | 14.3 |
| Xylitol 4% | 13.6 |
| Maltitol 2 % | 26.9 |
| Maltitol 4% | 32.6 |
| Erythritol 2 % | 29.2 |
| Erythritol 4% | 28.2 |

These results indicate that maltitol and erythritol are more effective than xylitol in masking the bitter taste of chlorhexidine.

### Example 2: Mouthwash

| | %w/w |
|---|---|
| Chlorhexidine digluconate | 0.12 |
| Maltitol | 3.0 |
| Ethanol 96% | 6.0 |
| Cremophor® RH60 | 0.20 |
| Flavour oil | 0.12 |
| Dye solution | 0.10 |
| Sodium saccharin | 0.01 |
| Water | to 100 |

### Example 3 Toothpaste

| | %w/w |
|---|---|
| Silica (abrasive) | 16.00 |
| Glycerin | 15.00 |
| Erythritol | 15.00 |
| Chlorhexidine digluconate | 1.00 |
| Poloxamer surfactant (Pluronic® F018) | 2.00 |
| Flavour | 1.00 |
| Dye solution (If striped) | 0.10 |
| Titanium dioxide | 0.70 |
| Sodium fluoride | 0.22 |
| Thaumatin | 0.02 |
| Sodium saccharin | 0.10 |
| Hydroxy ethyl cellulose | 2.4 |
| Water | to 100 |

## Claims

1. An oral hygiene composition comprising chlorhexidine or an orally acceptable addition salt thereof, a bitterness inhibitor selected from maltitol or erythritol or a mixture thereof, and an ionic fluorine-containing compound plus an orally acceptable carrier or excipient.

2. A composition according to claim 1 in which the chlorhexidine or an orally acceptable addition salt thereof is present in an amount from 0.005 to 10% by weight of the oral hygiene composition.

3. A composition according to claim 1 or 2 in which the chlorhexidine is present as the gluconate salt.

4. A composition according to any one of claims 1 to 3 in which the bitterness inhibitor is present in an amount from 0.1 to 30% by weight of the oral hygiene composition.

5. A composition according to any one of claims 1 to 4 in which the bitterness inhibitor is maltitol.

6. A composition according to any one of claims 1 to 5 further comprising a sweetening agent selected from saccharin and salts thereof, cyclamate salts, acesulfame, aspartame, thaumatin, and sugar alcohols such as sorbitol and xylitol, or a mixture thereof.

7. A composition according to any one of claims 1 to 7 further comprising an additional bitterness inhibitor selected from a sodium salt such as the chloride, bicarbonate, propionate, acetate, aspartate or gluconate, or a mixture thereof.

## Revendications

1. Composition d'hygiène orale contenant de la chlorhexidine ou un de ses sels d'addition, oralement acceptable, un inhibiteur d'amertume choisi parmi le maltitol ou l'érythritol ou un de leurs mélanges, et un composé contenant du fluor ionique plus un véhicule ou excipient oralement acceptable.

2. Composition selon la revendication 1, dans laquelle la chlorhexidine ou un de ses sels d'addition, oralement acceptable, est présente en quantité variant de 0,005 à 10 % en poids par rapport à la composition d'hygiène orale.

3. Composition selon la revendication 1 ou la revendication 2, dans laquelle la chlorhexidine est présente sous la forme saline de gluconate.

4. Composition selon l'une quelconque des revendications 1 à 3, dans laquelle l'inhibiteur d'amertume est présent en quantité variant de 0,1 à 30 % en poids par rapport à la composition d'hygiène orale.

5. Composition selon l'une quelconque des revendications 1 à 4, dans laquelle l'inhibiteur d'amertume est le maltitol.

6. Composition selon l'une quelconque des revendications 1 à 5, comprenant aussi un agent édulcorant choisi parmi la saccharine et ses sels, les sels cyclamates, l'acésulfame, l'aspartame, la thaumatine et les alcools sucres tels que le sorbitol et le xylitol ou un de leurs mélanges.

7. Composition selon l'une quelconque des revendications 1 à 7, comprenant aussi un inhibiteur d'amertume additionnel choisi sous la forme d'un sel de sodium tel que le chlorure, le bicarbonate, le propionate, l'acétate, l'aspartate ou le gluconate ou un mélange de ceux-ci.

## Patentansprüche

1. Mundhygienezusammensetzung, die Chlorhexidin oder ein oral akzeptables Salz davon, einen Bitterkeitsinhibitor, der aus Maltit oder Erythrit oder einer Mischung daraus ausgewählt ist, und eine ionische fluorhaltige Verbindung plus einen oral akzeptablen Träger oder Exzipienten umfasst.

2. Zusammensetzung gemäß Anspruch 1, worin das Chlorhexidin oder ein oral akzeptables Additionssalz davon in einer Menge von 0,005 bis 10 Gew.-% der Mundhygienezusammensetzung vorhanden ist.

3. Zusammensetzung gemäß Anspruch 1 oder 2, worin das Chlorhexidin als Gluconatsalz vorhanden ist.

4. Zusammensetzung gemäß einem der Ansprüche 1 bis 3, worin der Bitterkeitsinhibitor in einer Menge von 0,1 bis 30 Gew.-% der Mundhygienezusammensetzung vorhanden ist.

5. Zusammensetzung gemäß einem der Ansprüche 1 bis 4, worin der Bitterkeitsinhibitor Maltit ist.

6. Zusammensetzung gemäß einem der Ansprüche 1 bis 5, die ferner ein Süßungsmittel, das aus Saccharin und Salzen davon, Cyclamatsalzen, Acesulfam, Aspartam, Thaumatin und Zuckeralkoholen wie Sorbit und Xylit ausgewählt ist, oder eine Mischung daraus umfasst.

7. Zusammensetzung gemäß einem der Ansprüche 1 bis 7, die ferner einen zusätzlichen Bitterkeitsinhibitor umfasst, der aus einem Natriumsalz wie dem Chlorid, Bicarbonat, Propionat, Acetat, Aspartat oder Gluconat oder einer Mischung daraus ausgewählt ist.
